# EUROPÄISCHE PATENTSCHRIFT

(11) **EP 2 618 749 B1**
(45) Veröffentlichungstag und Bekanntmachung des Hinweises auf die Patenterteilung: **25.02.2015**
(21) Anmeldenummer: 11758463.1
(22) Anmeldetag: 16.09.2011
(51) Int. Cl.: A61B 17/122, A61B 17/128, A61B 18/14

(54) **CHIRURGISCHE CLIPANORDNUNG**
SURGICAL CLIP DEVICE
SYSTÈME D'AGRAFE CHIRURGICAL

(30) Priorität: 23.09.2010 DE 102010037748
(43) Veröffentlichungstag der Anmeldung: 31.07.2013
(73) Patentinhaber: Aesculap AG, 78532 Tuttlingen (DE)
(72) Erfinder: KELLER, Anton, 78589 Dürbheim (DE)
(74) Vertreter: Hoeger, Stellrecht & Partner Patentanwälte
(86) Internationale Anmeldenummer: PCT/EP2011/066125
(87) Internationale Veröffentlichungsnummer: WO 2012/038345

(56) Entgegenhaltungen:
- DE-A1- 2 639 956
- US-A- 5 201 900

## Beschreibung

Die Erfindung betrifft eine chirurgische Clipanordnung, insbesondere zum Verschließen von Gefäßen in der Neurochirurgie.

Zu diesem Zweck kommen in großem Umfang U- oder V-förmige metallische Clips zum Einsatz, die mit einer Clipanlegezange an dem Gefäß platziert und über die Schließkraft der Zange verformt das betroffene Gefäß verschließen. Solche Clips sind beispielsweise aus der DE 195 20 158 C2 bekannt.

Aus der US 2006/0217749 A1 ist ein Magazin mit chirurgischen Clips aus einem Kunststoffmaterial bekannt, in dem die aus Kunststoff gefertigten Clips in paralleler Ausrichtung zueinander gehalten sind.

Um den Verschluss eines Gefäßes durch einen Clip sicherer zu machen, wurden schon so genannte Doppelstegclips vorgeschlagen (vgl. z.B. DE 10 2006 001 344 A1), bei denen zwei einstückig miteinander ausgebildete, im Wesentlichen parallel zu einander angeordnete, U- oder V-förmige Bügel des Clips zwei in Längsrichtung des Gefäßes hintereinander liegende Verschlussstellen an dem Gefäß bilden.

Trotzdem kann es in kritischen Situationen noch vorkommen, dass auch ein solcher doppelter Verschluss des Gefäßes kleine Leckagen nicht verhindert, so dass immer noch kleinere Blutmengen austreten können.

Aufgabe der Erfindung ist es, eine chirurgische Clipanordnung vorzuschlagen, mit der das Verschließen von Gefäßen insbesondere in der Neurochirurgie noch sicherer gelingt.

Diese Aufgabe wird von einer chirurgischen Clipanordnung wie in Anspruch 1 definiert gelöst.

Die erfindungsgemäße chirurgische Clipanordnung setzt demnach nicht nur auf einen mechanischen Verschluss des Gefäßes, bei dem die beiden typischerweise metallischen Clipteile in Form von Klemmarmen das Gefäß in seinem Querschnitt zusammengedrückt halten und somit verschließen, sondern aufgrund der elektrisch voneinander isoliert vorliegenden Clipteile bzw. Klemmarme lässt sich gleichzeitig beim Anlegen der chirurgischen Clipanordnung ein Koagulations- bzw. Versiegelungsvorgang im Clipbereich initiieren, so dass neben dem mechanischen Verschluss noch ein stoffschlüssiger Verschluss des Gefäßes erzielt wird.

Darüber hinaus macht sich vorteilhaft bemerkbar, dass durch den Koagulations- und Versiegelungsvorgang eine Versteifung des Gewebes im Koagulationsbereich stattfindet, so dass die Clipanordnung zusätzlich einen sichereren Halt findet. Hierzu wird bevorzugt ein erfindungsgemäßer Clipapplikator verwendet der im Folgenden noch näher beschreiben wird.

Die chirurgische Clipanordnung mit ihren beiden voneinander elektrisch isoliert angeordneten elektrisch leitfähigen Clipteilen, insbesondere Klemmarmen, kann in vielfältigen Ausführungsformen realisiert werden.

Insbesondere sind die Clipteile Teil einer U- oder V-förmigen Clipkonfiguration, wobei die beiden Clipteile an einem ersten Ende an einem elektrisch isolierenden Halteteil gehalten sind und dabei die beiden Schenkel der U- oder V-förmigen Konfiguration bilden.

Bevorzugt ist das elektrisch isolierende Halteteil plastisch verformbar.

Kunststoffe sind typische Werkstoffe für die isolierenden Halteteile.

Insbesondere ist vorgesehen, dass die beiden Clipteile zueinander symmetrisch ausgebildet sind, wobei das elektrisch isolierende Halteteil so klein wie möglich gehalten wird, um für die Koagulation über die elektrisch leitfähigen Clipteile einen möglichst großen Wirkungsbereich bereitzustellen.

Insbesondere werden die beiden Clipteile im Wesentlichen identisch ausgebildet und spiegelbildlich mit ihrem ersten Ende an dem elektrisch isolierenden Halteteil fixiert angeordnet.

Alternativ kann das elektrisch isolierende Halteteil unsymmetrisch am U- oder V-förmigen Clip angeordnet sein, so dass ein erstes Clipteil oder Klemmarm den einen Schenkel und das mittlere Bogenteil umfasst, während das elektrisch isolierende Halteteil und das zweite Clipteil oder Klemmarm den anderen Schenkel bilden. Hier findet die plastische Verformung nicht zwingend am Halteteil, sondern vornehmlich am mittleren Bogenteil des einen Clipteils statt, der typischerweise aus Metall besteht.

Bei einer weiter bevorzugten Ausführungsform der erfindungsgemäßen chirurgischen Clipanordnung ist vorgesehen, dass die Clipteile bzw. Klemmarme an ihren dem ersten Ende entgegen gesetzten, jeweiligen freien Ende mit einer elektrisch isolierenden Verbindung miteinander verbindbar sind.

Die Verbindung wird bevorzugt als Rastverbindung ausgebildet.

Insbesondere werden dazu an den freien Enden der Clipteile elektrisch isolierende Rastelemente angeordnet, die beim mechanischen Schließen des Clips miteinander verrasten und so schon zu einer zusätzlichen mechanischen Fixierung des Clips an dem zu verschließenden Gefäß sorgen.

Alternativ zu der Rastverbindung kann auch vorgesehen sein, dass die elektrisch isolierende Verbindung eine sich an den freien Enden der Clipteile im Zuge der Wärmeentwicklung bei dem Koagulations- und Versiegelungsvorgang bildende stoffschlüssige Verbindung ist.

Bei einer anderen Ausführungsform der erfindungsgemäßen chirurgischen Clipanordnungen kann vorgesehen sein, dass zwei Clipteile, die jeweils im Wesentlichen U- oder V-förmig ausgebildet sind, parallel zueinander ausgerichtet auf einem vorgegebenen Abstand gehalten sind. Diese beiden Clipteile werden zum Applizieren an einem Gefäß von einem Clipapplikator erfasst, wobei dieser ein Greifwerkzeug für die beiden Clipteile mit voneinander elektrisch isolierten Greifbacken aufweist. Die beiden Clipteile lassen sich dann mit dem Clipapplikator an dem Gefäß applizieren, und im gleichen Schritt lässt sich über einen Koagulationsstrom ein Versiegelungsvorgang im Bereich des mechanischen Verschlusses des Gefäßes erzielen. Hierbei ergibt sich durch den Koagulationsvorgang ein zusätzlicher stoffschlüssiger Verschluss des Gefäßes.

Die beiden Clipteile können beispielsweise in einem Magazin, in einer gewünschten Anordnung bis zur Aufnahme durch das Greifwerkzeug vorgehalten werden. Die elektrisch gegeneinander isolierten Greifbacken halten die beiden Clipteile nachdem sie aus dem Magazin entnommen wurden bis zur Applikation elektrisch isoliert auf Abstand voneinander.

Die beiden Clipteile sind mittels einer elektrisch isolierenden Halterung parallel zueinander ausgerichtet auf dem vorgegebenen Abstand fest verbunden. Die Halterung kann wiederum aus einem Kunststoff hergestellt sein. Auch hier wird die Ausgestaltung der Greifbacken des Clipapplikators einfacher.

Die Halterung kann einteilig sein und beispielsweise die beiden Clipteile in ihrer jeweiligen Mitte miteinander verbinden.

Bei einer weiter bevorzugten Ausführungsform sind die freien Enden der beiden Clipteile jeweils miteinander mit einem elektrisch isolierenden Halteteil der Halterung verbunden.

Weiter bevorzugt können diese beiden Halteteile der Halterung dann auch als Rastelemente ausgebildet werden, die bei einer Applikation des Clips am Gefäß miteinander verrasten.

Wie bereits zuvor beschrieben, kann anstelle der Rastelemente auch vorgesehen sein, elektrisch isolierende Halteteile zu verwenden, die bei der Wärmeentwicklung bei der Koagulation und dem Versiegelungsvorgang des Gefäßes eine stoffschlüssige Verbindung miteinander eingehen.

Auch in diesem Fall sind dann die freien Clipenden miteinander fest verbunden, so dass neben der mechanischen Sicherung bei der Doppelclipanordnung wie oben beschrieben auch noch die Versiegelung über den Koagulationsvorgang im Gefäß hinzukommt.

Die Erfindung betrifft des Weiteren einen Clipapplikator zur Verwendung mit einer erfindungsgemäßen chirurgischen Clipanordnung wie oben beschrieben.

Der erfindungsgemäße Applikator weist ein Greifwerkzeug mit zwei elektrisch voneinander isolierten Greifbacken für die zwei Clipteile auf, wobei die Greifbacken mit einer einen Koagulationsstrom liefernden Stromquelle verbindbar sind.

Weiter bevorzugt ist, dass der Applikator ein Magazin mit einem Clipträger umfasst, wobei auf dem Clipträger mehrere chirurgische Clipanordnungen wie zuvor beschrieben angeordnet sind.

Die Stromquelle, mit der die Greifbacken des Applikators verbindbar sind, ist vorzugsweise eine Hochfrequenzstromquelle.

Diese und weitere Vorteile der Erfindung werden im Folgenden anhand der Zeichnungen noch näher erläutert.

Es zeigen im Einzelnen:
- Figuren 1A, B:: zwei Varianten einer ersten Ausführungsform einer erfindungsgemäßen chirurgischen Clipanordnung;
- Figuren 2A, B:: zwei Varianten einer zweiten Ausführungsform einer erfindungsgemäßen chirurgischen Clipanordnung;
- Figur 3:: einen Clipapplikator gemäß der vorliegenden Erfindung; und
- Figuren 4A bis D:: eine schematisch dargestellte Schrittfolge bei der Applikation einer Clipanordnung gemäß Figur 2A oder 2B.

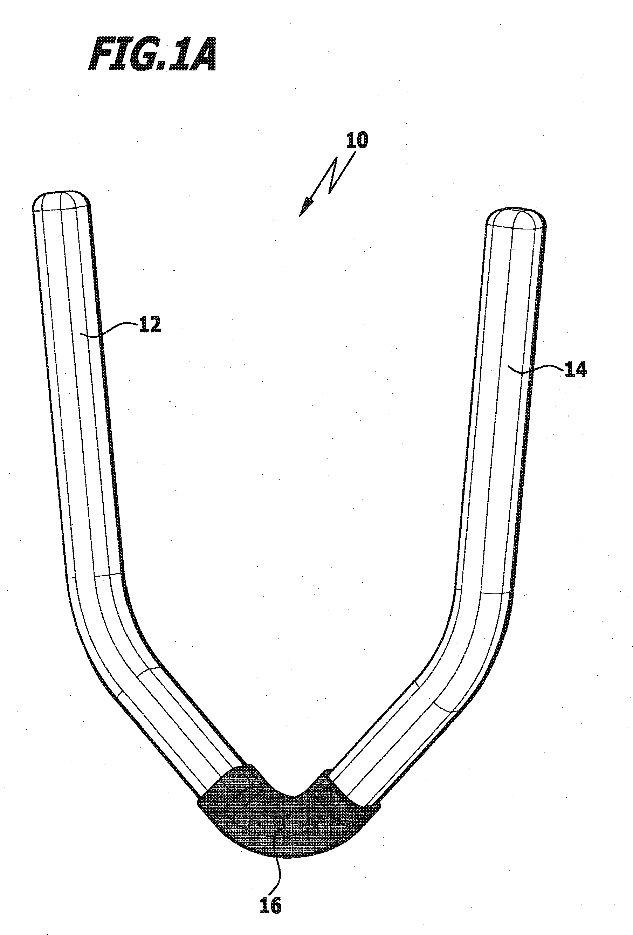

Figur 1A zeigt eine chirurgische Clipanordnung 10 (im Folgenden auch kurz Clip genannt) mit zwei elektrisch leitfähigen Clipteilen oder Klemmarmen in Form von Schenkeln 12 und 14, die in einer im Wesentlichen U-förmigen Anordnung elektrisch voneinander isoliert über ein Halteteil 16 in einem vorgegebenen Abstand zueinander und einander gegenüber liegend gehalten sind. Bei einer Applikation des Clips 10 an einem Gefäß werden die beiden Schenkel 12 und 14 von zwei Seiten des Gefäßes gegeneinander gepresst und bleiben durch das Halteteil 16 elektrisch voneinander isoliert. Das Halteteil wird aus einem plastisch verformbaren Kunststoff gefertigt, so dass sich beim Applizieren des Clips 10 ein permanenter mechanischer Verschluss des Gefäßes ergibt. In zusammengepresstem Zustand wird dann ein Koagulationsstrom über ein Greifwerkzeug eines Applikators einwirken gelassen, so dass neben dem mechanischen Verschluss des Gefäßes zusätzlich noch eine Versiegelung stattfindet.

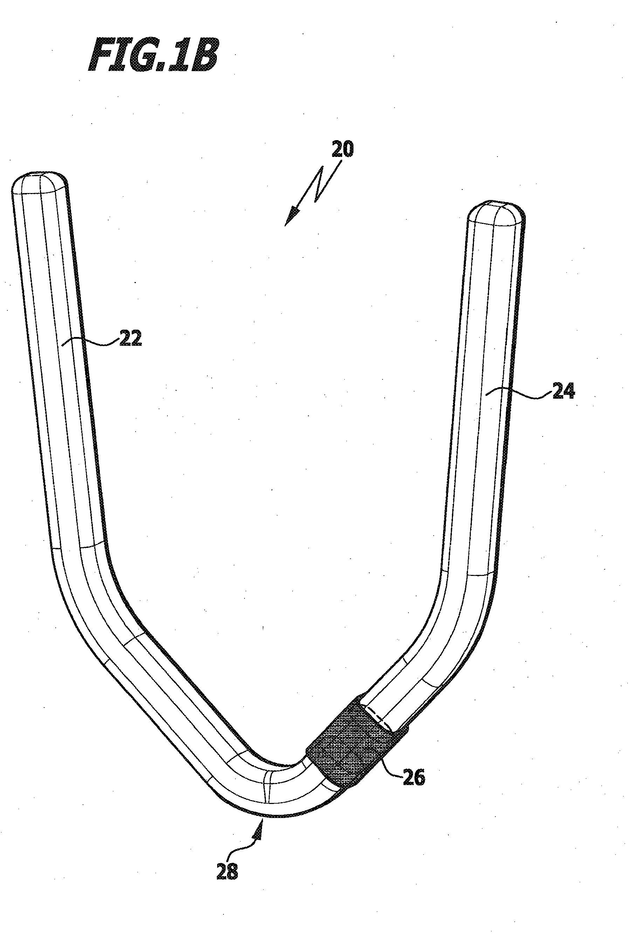

Die in Figur 1B dargestellte Clipanordnung 20 weist unsymmetrisch ausgebildete Klemmarme in Form der Schenkel 22, 24 und ein entsprechend außermittig angeordnetes elektrisch isolierendes Halteteil 26 auf.

Beim Applizieren des Clips 20 wird vornehmlich ein an den Schenkel 22 angeformtes Bogenteil 28 plastisch verformt und sorgt so für einen permanenten mechanischen Verschluss des Gefäßes.

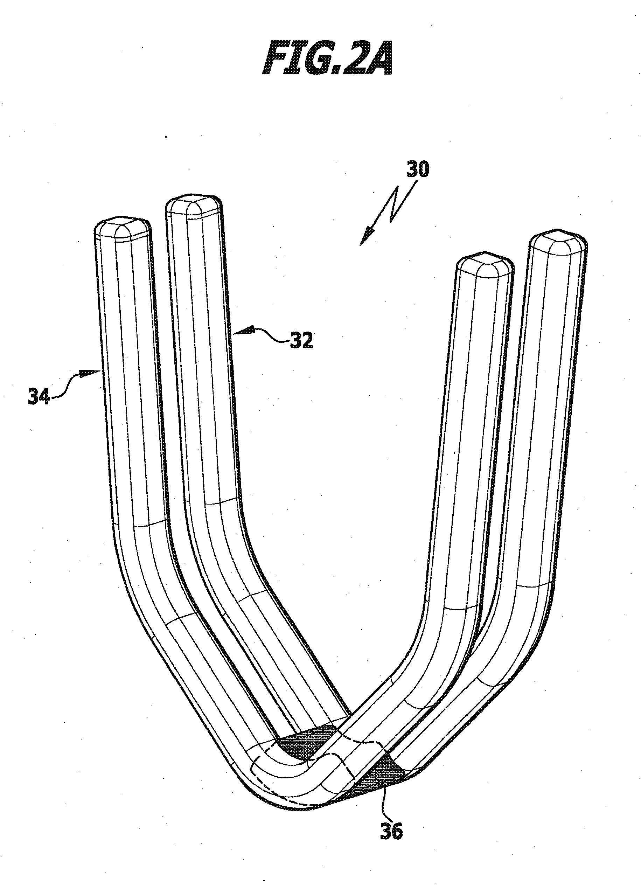

In Figur 2A ist eine chirurgische Clipanordnung 30 gezeigt, bei der zwei jeweils im Wesentlichen U-förmig ausgebildete Clipteile oder Klemmarme 32, 34 parallel zueinander angeordnet sind. Diese beiden Clipteile können über einen Steg 36 aus einem elektrisch isolierenden Material, z.B. Kunststoff, beispielsweise in ihrem mittleren Bereich miteinander verbunden und auf Abstand gehalten werden.

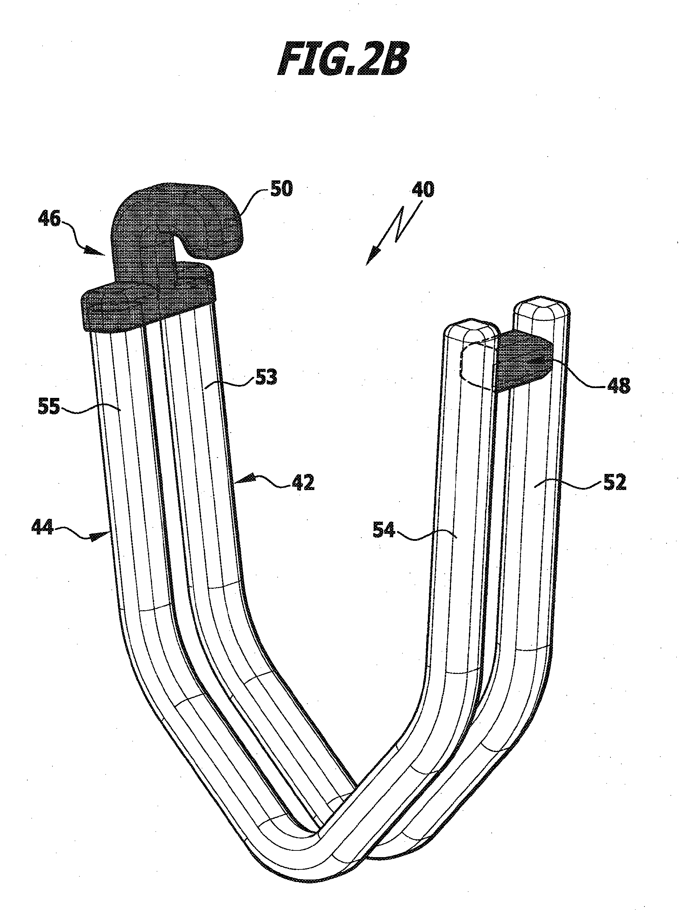

Weiter bevorzugt ist allerdings eine Clipanordnung 40 der Figur 2B, bei der zwei U-förmige Clipteile oder Klemmarme 42, 44 an ihren jeweiligen freien Enden 52,53; 54,55 über zwei Halteteile 46, 48 elektrisch isoliert auf Abstand gehalten sind, wobei das Halteteil 46 bevorzugt mit einem Rastelement 50 ausgerüstet ist, das in Figur 2B hakenförmig ausgebildet ist, und mit dem Halteteil 48 formschlüssig verbindbar ist.

Alternativ oder zusätzlich kann vorgesehen sein, dass das Material der Halteteile 46, 48 oder insbesondere auch des Rastteils 50 so ausgewählt ist, dass die sich bei der Koagulation des Gefäßmaterials entwickelnde Wärme ausreichend ist, um zu einer stoffschlüssigen Verbindung zwischen den Halteteilen 46, 48, insbesondere zwischen dem Teil 50 und dem Teil 48, zu führen.

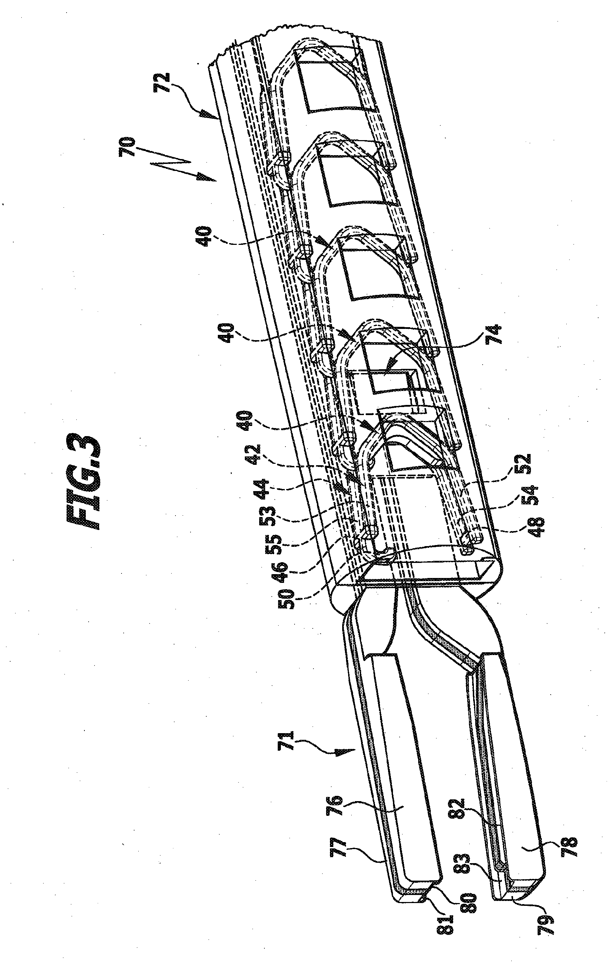

Figur 3 zeigt einen erfindungsgemäßen Clipapplikator 70, bei dem der Einfachheit halber der Bereich des Griffs zur Betätigung des Applikators 70 weggelassen ist.

Gezeigt ist in Figur 3 ein Schaft 72 des Applikators 70, in dem ein Clipmagazin 74 mit einer Reihe hintereinander angeordneter Clips 40 angeordnet ist. An seinem distalen Ende weist der Schaft 72 ein Greifwerkzeug 71 mit zwei elektrisch voneinander isoliert gehaltenen Greifbacken 76, 77 und 78, 79 auf, die bei einer Applikation eines Clips 40 dessen Clipteile 42, 44 mit deren freien Enden 52, 53; 54, 55 in Aufnahmenuten 80, 81; 82, 83 aufnehmen und führen.

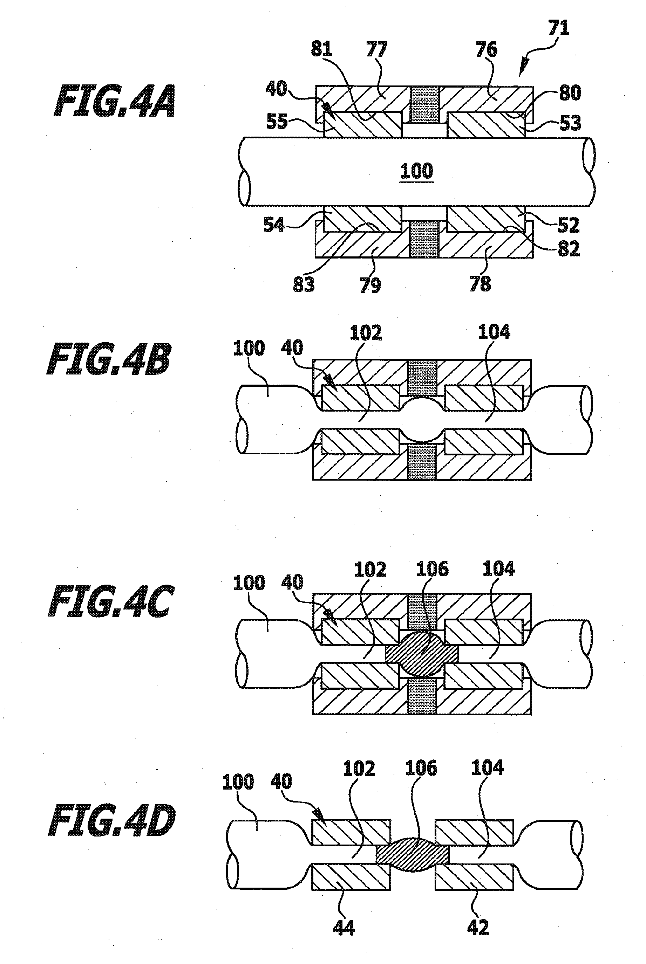

Sobald ein Clip 40 mit dem Greifwerkzeug 71 an einem Gefäß positioniert ist, können die Greifbacken 76, 77; 78, 79 mittels eines Handgriffs (nicht dargestellt) des Applikators 70 gegeneinander verschwenkt werden, so dass der Clip 40, wie dies im Zusammenhang mit der schematischen Darstellung der Figuren 4A bis 4D noch näher erläutert wird, zusammengepresst wird, wodurch sich zunächst bei dem Gefäß ein doppelter mechanischer Verschluss ergibt.

Aufgrund der elektrisch isolierten Halterung der Greifbacken 76, 77; 78, 79 des Clipapplikators 70 lassen sich diese dann bestromen, wobei über den körperlichen Kontakt der freien Enden der Schenkel 52, 53; 54, 55 des Clips 40 mit den Greifbacken 76, 77; 78, 79 auch ein elektrisch leitfähiger Kontakt hergestellt ist, so dass die Schenkel des Clips 40 bestromt werden können.

Die Schrittfolge bei der Applikation einer erfindungsgemäßen Clipanordnung am Beispiel der erfindungsgemäßen Clipanordnung 40 der Figur 2B ist in den Figuren 4A bis D schematisch dargestellt. Analog stellt sich der Vorgang mit Clips der Figuren 1A, 1B und 2A dar.

Figur 4A zeigt den Clip 40 in Schnittdarstellung mit seinen Schenkeln 52, 54 unterhalb und den Schenkeln 53, 55 oberhalb eines Gefäßes 100. Die Schenkel 52, 54 sind in Nuten 82, 83 der Greifbacken 78, 79 geführt und gehalten, und die Schenkel 53, 55 sind in den Nuten 80, 81 der Greifbacken 76, 77 geführt und gehalten.

Im nächsten Schritt werden, wie in Figur 4B dargestellt, die Greifbacken 76, 77 und 78, 79 gegeneinander bewegt und dabei die Schenkel 52, 54 und 53, 55 einander angenähert und dabei das Gefäß 100 in den in Längsrichtung des Gefäßes beabstandeten Bereichen 102 und 104 mechanisch geschlossen.

In der geschlossenen Stellung des Clips 40 rastet dann das Teil 50 an dem Halteteil 48 ein und sichert so die geschlossene Stellung des Clips 40.

Nachfolgend werden die Greifbacken 76, 78 und 77, 79 paarweise mit einem Pol einer HF-Stromquelle verbunden, so dass sich dann zwischen den mechanisch verschlossenen Bereichen 102, 104 des Gefäßes 100 ein koagulierter Bereich 106 des Gefäßes 100 bildet (vgl. Figur 4C).

Sobald der Koagulationsvorgang abgeschlossen ist, was typischerweise nur wenige Sekunden/den Bruchteil einer Sekunde benötigt, werden die Greifbacken 76, 77; 78, 79 wieder in ihre Ausgangsstellung zurückgefahren und der Applikator 70 vom Gefäß 100 entfernt. Zurück bleibt das Gefäß 100 mit den Schenkeln 52, 53 und 54, 55 des applizierten und über die Halteteile 46, 48, 50 mechanisch verriegelten Clips 40, wobei zusätzlich zu dem doppelten mechanischen Verschluss des Gefäßes 100 an den Bereichen 102, 104 ein koagulierter Bereich 106 für zusätzliche Sicherheit sorgt (Figur 4D).

## Patentansprüche

1. Chirurgische Clipanordnung (30; 40), insbesondere zum Verschließen von Gefäßen in der Neurochirurgie, mit mindestens zwei von einander elektrisch isoliert in einem vorgegebenen Abstand einander gegenüber liegend angeordneten elektrisch leitfähigen Clipteilen (32, 34; 42, 44), welche mittels einer elektrisch isolierenden Halterung parallel zueinander ausgerichtet auf einem vorgegebenen Abstand gehalten und fest verbunden sind, wobei die zwei Clipteile (32, 34; 42, 44) jeweils eine im Wesentlichen U- oder V-förmige Konfiguration aufweisen.

2. Chirurgische Clipanordnung (30; 40) nach Anspruch 1, **dadurch gekennzeichnet, dass** die Halterung ein erstes Halteteil (36; 46; 48) umfasst, welches die beiden im Wesentlichen U- oder V-förmig ausgebildeten Clipteile (32, 34; 42, 44) fest miteinander verbindet.

3. Chirurgische Clipanordnung (40) nach Anspruch 2, **dadurch gekennzeichnet, dass** die Halterung ferner ein zweites Halteteil (48) umfasst, wobei das erste Halteteil (46) zwei benachbarte freie Enden der Clipteile (42, 44) verbindet und das zweite Halteteil (48) zwei weitere benachbarte freie Enden der U- oder V-förmigen Clipteile (42, 44) fest miteinander verbindet.

4. Chirurgische Clipanordnung (40) nach Anspruch 3, **dadurch gekennzeichnet, dass** die beiden Halteteile (46, 48) der Haltevorrichtung miteinander verbindbar sind.

5. Chirurgische Clipanordnung (40) nach Anspruch 4, **dadurch gekennzeichnet, dass** die beiden Halteteile (46, 48) lösbar miteinander verbindbar sind.

6. Chirurgische Clipanordnung nach Anspruch 4 oder 5, **dadurch gekennzeichnet, dass** die beiden Halteteile (46, 48) rastend miteinander verbindbar sind.

7. Clipapplikator (70) zur Verwendung mit der chirurgischen Clipanordnung (30; 40) nach einem der Ansprüche 1-6, **dadurch gekennzeichnet, dass** der Applikator ein Greifwerkzeug mit elektrisch voneinander isolierten Greifbacken für die zwei Clipteile (32, 34; 42, 44) aufweist und dass die Greifbacken mit einer einen Koagulationsstrom liefernden Stromquelle verbindbar sind.

8. Clipapplikator (70) nach Anspruch 7, **dadurch gekennzeichnet, dass** der Applikator ein Magazin (74) mit einem Clipträger umfasst, wobei auf dem Clipträger mehrere chirurgische Clipanordnungen gemäß einem der Ansprüche 1 bis 6 angeordnet sind.

9. Clipapplikator (70) nach Anspruch 7 oder 8, **dadurch gekennzeichnet, dass** die Stromquelle eine Hochfrequenzstromquelle ist.

## Claims

1. Surgical clip device (30; 40), in particular for closing vessels in neurosurgery, comprising at least two electrically conductive clip parts (32, 34; 42, 44) arranged in electrical isolation from each other and spaced apart at a predetermined distance opposite each other, said clip parts (32, 34; 42, 44) being fixedly connected in parallel alignment with each other and spaced apart at the predetermined distance by an electrically isolating holder, wherein the two clip parts in each case have a substantially U-shaped or V-shaped configuration.

2. Surgical clip device (30; 40) in accordance with claim 1, wherein the holder comprises a first holding part (36; 46; 48) which fixedly connects the two clip parts (32, 34; 42, 44) of substantially U-shaped or V-shaped configuration.

3. Surgical clip device (30; 40) in accordance with claim 2, wherein the holder further comprises a second holding part (48), wherein the first holding part (46) connects two adjacent free ends of the clip parts (42, 44) and the second holding part (48) fixedly connects two further adjacent free ends of the U-shaped or V-shaped clip parts (42, 44).

4. Surgical clip device (40) in accordance with claim 3, wherein the two holding parts (46, 48) of the holding device are connectable to each other.

5. Surgical clip device (40) in accordance with claim 4, wherein the two holding parts (46, 48) are releasably connectable to each other.

6. Surgical clip device in accordance with claim 4 or 5, wherein the two holding parts (46, 48) are latchingly connectable to each other.

7. Clip applicator (70) for use with a surgical clip device (30; 40) in accordance with any one of claims 1 to 6, **characterized in that** the applicator comprises a gripping tool having mutually electrically isolated gripping jaws for the two clip parts (32, 34; 42, 44) and wherein the gripping jaws are connectable to a coagulation current-delivering current source.

8. Clip applicator (70) in accordance with claim 7, wherein the applicator comprises a magazine (74) having a clip carrier, wherein the clip carrier has arranged thereon a plurality of surgical clip devices in accordance with any one of claims 1 to 6.

9. Clip applicator (70) in accordance with claim 7 or 8, wherein the current source is a high-frequency current source.

## Revendications

1. Système d'agrafe ou de clip chirurgical (30; 40), notamment pour fermer des vaisseaux en neurochirurgie, comprenant au moins deux parties de clip (32, 34; 42, 44) électriquement conductrices, agencées de manière mutuellement opposée et espacée selon une distance prédéterminée, et de façon à être mutuellement isolées sur le plan électrique, un élément de maintien électriquement isolant maintenant et reliant de manière fixe ces parties de clip de façon à ce qu'elles soient orientées parallèlement les unes aux autres et espacées d'une distance prédéterminée, les deux parties de clip (32, 34; 42, 44) présentant chacune une configuration sensiblement en forme de U ou de V.

2. Système de clip chirurgical (30; 40) selon la revendication 1, **caractérisé en ce que** l'élément de maintien comprend une première pièce de maintien (36; 46; 48), qui relie de manière fixe les deux parties de clip (32, 34; 42, 44) configurés sensiblement en forme de U ou de V.

3. Système de clip chirurgical (40) selon la revendication 2, **caractérisé en ce que** l'élément de maintien comprend, par ailleurs, une deuxième pièce de maintien (48), la première pièce de maintien (46) reliant deux extrémités libres voisines des parties clip (42, 44), et la deuxième pièce de maintien (48) reliant de manière fixe deux autres extrémités libres voisines des parties de clip (42, 44) en forme de U ou de V.

4. Système de clip chirurgical (40) selon la revendication 3, **caractérisé en ce que** les deux pièces de maintien (46, 48) du dispositif de maintien peuvent être reliées l'une à l'autre.

5. Système de clip chirurgical (40) selon la revendication 4, **caractérisé en ce que** les deux pièces de maintien (46, 48) peuvent être reliées de manière amovible l'une à l'autre.

6. Système de clip chirurgical selon la revendication 4 ou la revendication 5, **caractérisé en ce que** les deux pièces de maintien (46, 48) peuvent être reliées l'une à l'autre par encliquetage.

7. Applicateur de clip (70) destiné à être utilisé avec le système de clip chirurgical (30; 40) selon l'une des revendications 1 à 6, **caractérisé en ce que** l'applicateur comprend un outil de préhension comportant des mors de préhension isolés électriquement les uns des autres pour les deux parties de clip (32, 34; 42, 44), et **en ce que** les mors de préhension peuvent être reliés à une source de courant fournissant un courant de coagulation.

8. Applicateur de clip (70) selon la revendication 7, **caractérisé en ce que** l'applicateur comprend un magasin (74) avec un support de clips, plusieurs systèmes de clips chirurgicaux selon l'une des revendications 1 à 6 étant agencés sur le support de clips.

9. Applicateur de clip (70) selon la revendication 7 ou la revendication 8, **caractérisé en ce que** la source de courant est une source de courant haute-fréquence.
